(19) Europäisches Patentamt
European Patent Office
Office européen des brevets

(11) Publication number: **0 361 510 B1**

# (12) EUROPEAN PATENT SPECIFICATION

(45) Date of publication of patent specification: **01.12.93**

(51) Int. Cl.$^5$: **C07D 407/14**, A61K 31/35

(21) Application number: **89118062.2**

(22) Date of filing: **29.09.89**

## (54) Antitumor substance, process for preparing the same, and anticancer agent containing the same.

(30) Priority: **30.09.88 JP 244252/88**

(43) Date of publication of application:
**04.04.90 Bulletin 90/14**

(45) Publication of the grant of the patent:
**01.12.93 Bulletin 93/48**

(84) Designated Contracting States:
**AT BE CH DE ES FR GB IT LI LU NL SE**

(56) References cited:
**EP-A- 0 326 173**

**HELVETICA CHIMICA ACTA, vol. 62, no. 8, December 1979, pages 2525-2533, Schweizerische Chemische Gesellschaft, Basel, CH; M. ZEHNDER et al.: "The structure of the antibiotic hedamycin. V. Crystal structure and absolute configuration"**

(73) Proprietor: **MEIJI SEIKA KAISHA LTD.**
**4-16 Kyobashi 2-chome**
**Chuo-ku Tokyo(JP)**

(72) Inventor: **Sato, Yoshikazu Meiji Seika Kaisha, Ltd. Pharmac.**
**Research Lab.**
**760, Morooka-cho**
**Kohoku-ku**
**Yokohama-shi Kanagawa(JP)**
Inventor: **Yamamoto, Haruo Meiji Seika Kaisha, Ltd. Pharmac.**
**Research Lab.**
**760, Morooka-cho**
**Kohoku-ku**
**Yokohama-shi Kanagawa(JP)**
Inventor: **Inoye, Shigeharu Meiji Seika Kaisha, Ltd. Pharmac.**
**Research Lab.**
**760, Morooka-cho**
**Kohoku-ku**
**Yokohama-shi Kanagawa(JP)**
Inventor: **Nakazawa, Tadashi Meiji Seika Kaisha, Ltd. Pharmac**
**Research Lab.**
**760, Morooka-cho**
**Kohoku-ku**
**Yokohama-shi Kanagawa(JP)**
Inventor: **Sezaki, Masaji Meiji Seika Kaisha, Ltd. Pharmac**
**Research Lab.**
**760, Morooka-cho**
**Kohoku-ku**
**Yokohama-shi Kanagawa(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

(74) Representative: **WILHELMS, KILIAN & PARTNER Patentanwälte**
**Eduard-Schmid-Strasse 2**
**D-81541 München (DE)**

## Description

This invention relates to compounds having an antitumor activity, a process for preparing the same, and an anticancer agent containing the same or a pharmaceutically acceptable salt thereof as an active ingredient.

SF2587 substance is an antibiotic isolated from the culture of a strain of actinomycetes belonging to the genus Streptomyces (JP-A-1-193265) and is represented by formula (II):

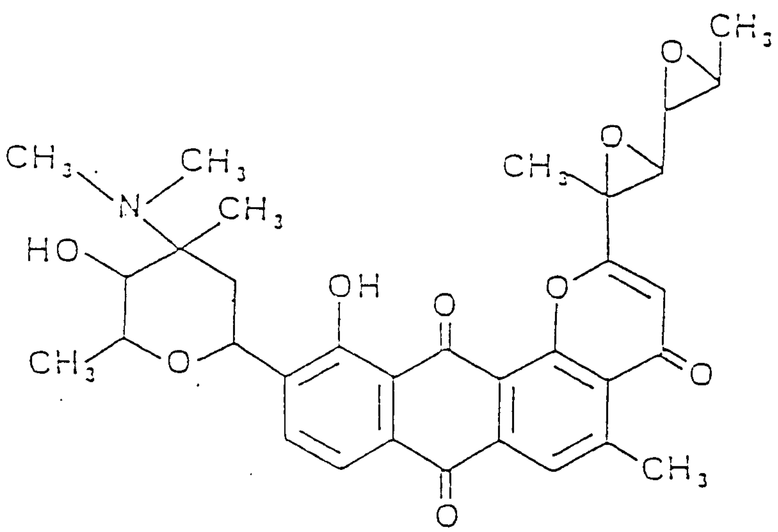

(II)

SF2587 exhibits considerable inhibitory activity on P388 tumor cells and revealed excellent therapeutic effects in experiments performed on mice transplanted with P388 tumor cells. Nevertheless, since SF2587 substance is of relatively high toxicity ($LD_{50}$: about 5 mg/kg, mouse, i.v.).

## SUMMARY OF THE INVENTION

An object of the present invention is to provide deritatives of SF2587 which have reduced toxicity and enhanced antitumor activity as compared with the SF2587 substance.

The inventors have conducted extensive studies on a method of reducing the toxicity of SF2587 while further improving its antitumor activity. As a result, it has now been found that the above object can be attained by an acyl derivative of SF2587 obtained by chemical conversion of SF2587 or a pharmaceutically acceptable salt thereof, said acyl derivative being represented by formula (I):

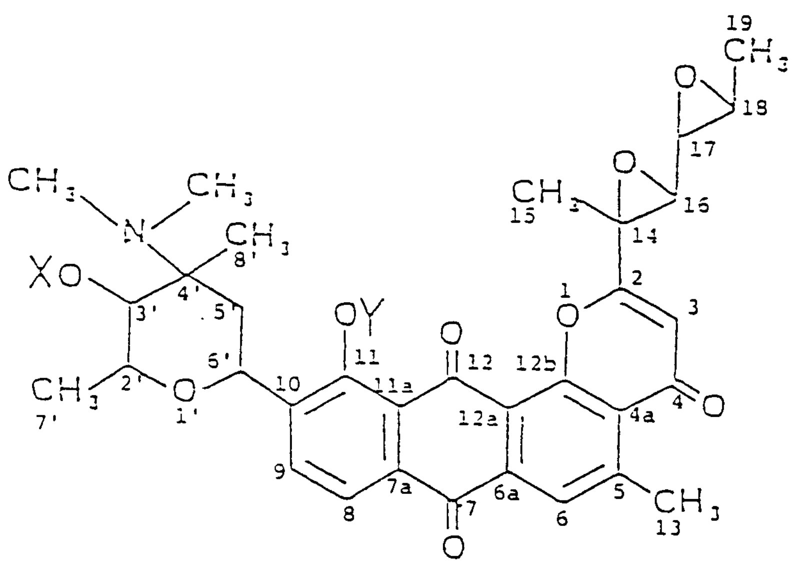

(I)

wherein X and Y each represents a hydrogen atom or a group RCO-, wherein R represents an alkyl group having from 1 to 4 carbon atoms, provided that X and Y do not simultaneously represent a hydrogen atom.

The present invention further relates to an anticancer agent containing a SF2587 derivative represented by formula (I) or a pharmaceutically acceptable salt thereof as an active ingredient in an amount effective for treating cancer.

The present invention furthermore relates to a process for preparing a SF2587 derivatives represented by formula (I), which comprises reacting SF2587 represented by formula (II)

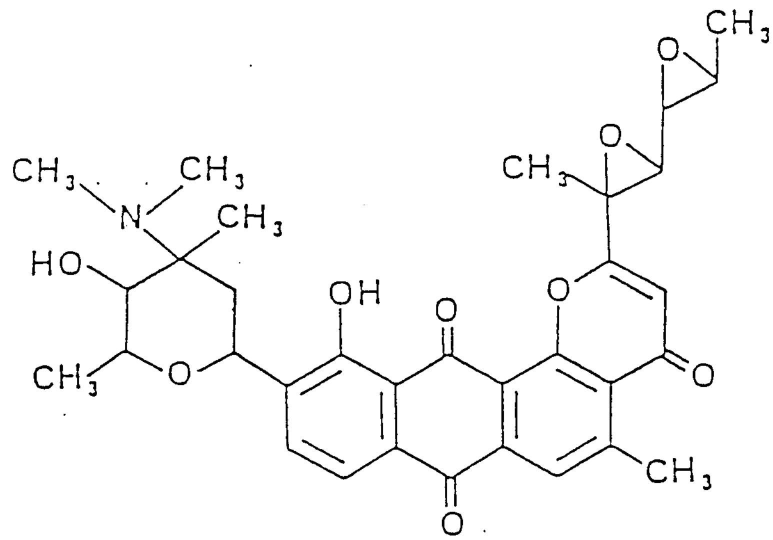

(II),

with an acylating agent.

## DETAILED DESCRIPTION OF THE INVENTION

In formula (I), examples of the group represented by RCO- includes an acetyl group, an n-propionyl group, an n-butyryl group, an isobutyryl group and an n-valeryl group. Among these, an n-propionyl group, an n-butyryl group, an isobutyryl group and an n-valeryl group are particularly preferred.

SF2587 can be prepared, for example, by cultivating a microorganism capable of producing the substance and recovering it from the culture.

An example of a SF2587-procuding strain is strain SF2587 which has been deposited at the Fermentation Research Institute of Japan under deposit No. BP-2244 in accordance with the Budapest treaty.

The bacteriological characteristics of strain SF2587 are as follows.

### I. Morphology

The vegetative mycelium is long-extending and well branched, and is not fragmented under usual conditions. The aerial mycelium is abundant, with good sporulation, on oatmeal agar, inorganic salts-starch agar, yeast extract-malt extract agar, etc. The aerial mycelium is monopodially branched, with no apparent whirl formation. The spore chain at the terminal end of the aerial mycelium is mostly spiral but at times undulating. Electron microscopy reveals that the spore is elipsoidal to cylindrical and measures 0.5 to 0.9 × 0.7 to 1.3 μm. The spore wall ornamentation is smooth. Usually 20 or more spores occur in chains. the sporangium, motile spore, sclerotium, etc. are not observed.

### II. Cultural characteristics

The cultural characteristics of strain SF2587 on various media are shown in Table 1. In the description of color, the color standards given in parentheses are those used in Container Corporation of America's Color Harmony Manual. The obsevation was made after 14-21 days of incubation at 28°C.

Table 1

| Medium | Growth (reverse color) | Aerial mycerium | Soluble pigment |
|---|---|---|---|
| Sucrose nitrate agar | Good, topas (3ne) | Abundant, gray (2fe) | None |
| Glucose asparagine agar | Poor-fair, light yellow (2ec) | None | None |
| Glycerol asparagine agar | Fair, colorless | Fair, gray (3fe) | None |
| Calcium malate agar | Fair, gray yellow (2ne) | Abundant, gray (3fe) | None |
| Inorganic salts-starch agar | Good, rose-beige (4ge) | Abundant, gray (3fe) | None |
| Oatmeal agar | Good, rose-beige (4ge) | Abundant, gray (3fe) | None |
| Yeast extract-malt extract agar | Good, light brown (31g) | Abundant, gray (3fe) | Pale yellow |
| Tyrosine agar | Fair, tan (3ie) | Abundant, gray (3fe) | None |
| Nutrient agar | Fair, topas (3ne) | Sparse, white | None |
| Bennett agar | Fair, gray yellow (2gc) | Fair, gray (3fe) | None |

III. Physiological characteristics

(1) Temperature range for growth: On yeast extract-malt extract agar, growth occurs in the temperature range of 15-45 °C and good growth at 26-37 °C.
(2) Liquefaction of gelatin: Positive
(3) Hydrolysis of starch: Positive
(4) Reduction of nitrate: Negative
(5) Peptonization of skimmed milk: Positive Coagulation of skimmed milk: Positive
(6) Salt resistance: Grwoth occurs in media containing 10% NaCl but does not in media containing 12% or more of NaCl.
(7) Production of melanoid pigment: Negative

IV. Utilization of carbon sources (ISP No. 9 medium)

All of D-glucose, glycerol, D-xylose, L-arabinose, L-rhamnose, D-mannitol, D-fructose, raffinose, myoinositol and sucrose are well assimilated.

V. Cell wall composition

As analyzed by the method of Becker et al. (Appl. Microbiol., 13, 236 (1965)), the cell wall fraction contains LL-diaminopimellic acid.

Thus, strain SF2587 is considered to belong to the genus Streptomyces, which is among actinomycetes, with aerial mycelium in the Gray color series, the terminal end of aerial mycelium being mostly spiral, a smooth spore surface, and a reverse color of gray yellow with a tinge of red, and does not produce melanoid pigments.

Like other actinomycetes, strain SF2587 is liable to undergo variation in characteristics. For example, mutant strains (spontaneous or induced) as well as transductants and transformants (genetically engineered) of, or derived from, strain SF2587 can also be used for the purposes of this invention only if they are able to produce SF2587 sutstance. In the method of this inveniton, the above-mentioned strain is cultivated in a medium containing the ordinary nutrients which microorganisms may utilize. Thus, as nutrient sources, those known sources which have been conventionally utilized in the culture of actinomycetes can be utilized. For example, as carbon sources, use can be made of glucose, glucose or maltose syrup, dextrin, starch, sucorse, molasses, and animal or vegetable oils, preferably maltose syrup, starch, glucose, soybean oil and sucrose. As nitrogen sources, used can be made of soybean meal, wheat germs, corn steep liquor, cottonseed meal, meat extract, peptone, yeast extract, ammonium sulfate, sodium nitrate, urea and so on, preferably soybean meal and Pharmamedia (trade name of cottonseed meal produced by Troders Oil Mill Co., Texas). In addition, it is sometimes advantageous to incorporate various inorganic salts

capable of providing sodium, calcium, magnesium, cobalt, chlorine, phosphate, sulfate and other ions. Preferable examples of inorganic salts include $CaCO_3$, $FeSO_4 \cdot 7H_2O$ and $CoCl_2 \cdot 6H_2O$. It is also appropriate to add suitable amounts of organic and/or inoragnic substances which assist in the growth of the microorganism and promote production of SF2587. A preferable example thereof is distiller's solubles.

The pH value of the medium preferably ranges from 6.0 to 7.5.

To grow the strain, aerobic culture is carried out, and submerged aerobic culture is particularly advantageous. While the incubation temperature may range from 26 to 37°C, it is appropriate to carry out the cultivation at about 28°C in many instances. Though it depends on the medium and cultural conditions used, accmulation of SF2587 substance reaches a peak generally in 2 to 7 days, whether in shake culture of in tank culture (preferably tank culture). When the accumulation of SF2587 substance has become maximal, the incubation is stopped and the desired substance is isolated and purified from the resulting culture.

Since SF2587 is fat-soluble, this property can be exploited in its isloation and purification from the culture broth. Thus, there can be advantageously utilized methods of column chromatography using synthetic adsorbents such as Amberlite XAD-2 (Rhom & Haas Co.), Diaion HP-20 (Mitsubishi Kasei Corporation), gel filtration aids such as Sephadex LH-20 (Pharmacia Fine Chemicals), Toyoperal HW-40 (Tosoh Corporation), silica gel, alumina, or solvent extraction processes using ethyl acetate, chloroform and so on.

By any or a suitable combination of these techniques, SF2587 can be islolated in high purity.

The compound of formula (I) according to the present invention can be prepared by acylating SF2587 with an acylating agent in the presence of a base. The acylating agent which can be used in this invention includes acetic anhydride, acetyl chloride, propionic anhydride, propionyl chloride, n-butyric anhydride, n-butyryl chloride isobutyryl chloride, isobutyric anhydride, n-valeryl chloride and n-valeric anhydride. The reaction is carried out at -10°C to 100°C for 5 to 50 hours, preferably 5 to 50°C for 24 to 48 hours. The base to be used for obtainining a monoacylated compound includes sodium acetate, and that for obtaining a diacylated compound includes pyridine, dimethylaminopyridine, and triethylamine. The molar ratio of the acylating agent and the base to SF2587 ranges from 30 to 70, 140 to 160, respectively.

The compound of formula (I) can be purified from the reaction mixture by silica gel column chromatography, followed by recrystallization if required.

Examples of a pharmaceutically acceptable salt of the compound of formula (I) according to the present invention include hydrochloride, sulfate, phosphate, acetate, citrate, malonate, salicylate, maleate, fumarate, succinate, ascorbate, malate, methanesulfonate, lactate, gluconate, glucronate, amidosulfonate, benzoate and tatrate.

The anticancer agent containing the compound of formula (I) is administered orally or parenterally. When parenterally given, the compound is dissolved in physiological saline or an appropriate buffer solution and formulated into a solution or a suspension for injection, rectal infusion or local application. When orally administered, the compound of formula (I) is mixed with pharmaceutically acceptable vehicles, and the like and, if desired, formulated into gelatin capsules, tablets, etc. each containing 1 to 200 mg of the active ingredient. A suitable dose to mammals inclusive of humans ranges from 0.5 to 40 mg/kg-b.w. one to three times per day.

The present invention is now illustrated in greater detail by way of the following Examples.

## EXAMPLE 1

### Synthesis of 3′-O-acetyl SF2587 Substance (Compound 1)

In 3 mℓ of acetic anhydride was dissolved 40 mg of SF2587, and 24.9 mg of sodium acetate was added thereto while stirring at 20°C. The stirring at 20°C was further continued for an additional period of 2 hours. The reaction mixture was poured into 50 mℓ of ice-water, neutralized with a 0.1 N sodium hydroxide aqueous solution, and extracted twice with 20 mℓ portions of chloroform. The chloroform layer was separated and dried over anhydrous sodium sulfate. After removal of the sodium sulfate by filtration, the resulting chloroform solution was concentrated under reduced pressure to obtain an orange solid. This crude product was purified by silica gel column chromatography using a 50/1 (by volume) mixed solvent of chloroform/methanol to obtain 29.7 mg of an orange 3-0′-acetyl derivative of SF2587 [Compound 1: the compound of formula (I) wherein X = $CH_3CO$-; Y = H.

Melting Point: 125-129°C

[α] = +172.2° (c=0.1, chloroform)

EI-MS: m/z 631 ($M^+$)

UV λ nm(ε): 243(44550), 260(sh., 27760), 422(9020)
IR ν $cm^{-1}$: 3450, 1740, 1660, 1635, 1590
$^{1}H$-NMR ($CDCl_3$, ppm): 13.32 s, 8.07 s, 7.94 d, 7.83 d, 6.47 s, 5.53 dd, 5.16 d, 4.32 dq, 3.36 d, 3.12 dq, 2.99 s, 2.89 dd, 2.50 d, 2.38 d, 2.20 s, 1.95 s, 1.74 s, 1.51 d, 1.45 d, 0.98 s
$^{13}C$-NMR ($CDCl_3$, ppm): 187.6, 181.5, 178.8, 170.5, 166.2, 159.2, 156.3, 149.9, 140.7, 136.3, 133.3, 130.6, 126.5, 126.0, 119.8, 119.5, 115.9, 110.1, 76.7, 70.4, 64.2, 63.9, 57.7, 57.6, 55.5, 51.9, 42.3, 39.5, 24.2, 21.3, 17.3, 14.7, 14.5, 13.9

## EXAMPLE 2

### Synthesis of 3′, 11-O-acetyl-SF2587 (Compound 2)

In 4 mℓ of pyridine was suspended 100 mg of SF2587, and 2 mℓ of acetic anhydride was added dropwise to the suspension while stirring under ice-cooling. The mixture was stirred for 30 minutes under ice-cooling and then at 20 °C for 3 hours. The reaction mixture was poured into 20 mℓ of ice-water and then extracted twice with 20 mℓ portions of chloroform. The chloroform layer was separated, washed with 20 mℓ of a saturated sodium chloride aqueous solution, and dried over anhydrous sodium sulfate. After removal of the sodium sulfate by filtration, the resulting chloroform solution was concentrated under reduced pressure to obtain a yellow oily substance. The oily substance was dried under reduced pressure overnight and purified by silica gel column chromatography using a 100/1 (by volume) mixed solvent of chloroform/methanol to obtain 64.3 mg of a yellow 3′, 11-O-diacetyl derivative of SF2587 [Compound 2: the compound of formula (I) wherein X = $CH_3CO$-; Y = $CH_3CO$-].
Melting Point: 128-132 °C
[α] = +42.1 ° (c = 0.1, chloroform)
EI-MS: m/z 673 ($M^+$)
UV λ nm(ε): 241(32740), 258(30860), 363(7370)
IR ν $cm^{-1}$: 3450, 1770, 1750, 1680, 1650, 1590
$^{1}H$-NMR ($CDCl_3$, ppm): 8.21 d, 8.04 d, 7.99 s, 6.48 s, 5.32 m, 5.24 d, 4.32 d, 3.49 d, 3.14 dd, 2.97 s, 2.87 dd, 2.56 s, 2.38 m, 2.30 s, 2.20 s, 1.90 s, 1.57 m, 1.43 d, 1.38 d, 1.07 d
$^{13}C$-NMR ($CDCl_3$, ppm): 181.9, 180.5, 179.0, 174.2, 170.4, 165.6, 155.7, 148.6, 146.1, 144.8, 135.3, 132.6, 132.3, 126.4, 125.7, 125.4, 125.2, 121.6, 110.8, 75.3, 70.6, 64.5, 63.5, 58.2, 57.3, 55.6, 51.7, 42.4, 38.7, 23.9, 21.2, 21.1, 17.2, 14.7, 14.4, 14.2

## EXAMPLE 3

### Synthesis of 3′-O-propionyl SF2587 (Compound 3)

In 2 mℓ of propionic anhydride was suspended 22.3 mg of SF2587, and 15.5 mg of anhydrous sodium acetate was added to the suspension at 20 °C while stirring. The stirring at 20 °C was further continued for an additional period of 4 hours. The reaction mixture was poured into 15 mℓ of ice-water, neutralized with a 0.1N sodium hydroxide aqueous solution, and extracted twice with 10 mℓ portions of chlorofrom. The chloroform layer was separated and dried over anhydrous sodium sulfate. After removing the sodium sulfate by filtration, the resulting chloroform solution was concentrated under reduced pressure and then purified by silica gel column chromatography using a chloroform/methanol (50:1 by volume) mixed solvent as an eluent to obtain 21.9 mg of a yellowish brown 3′-O-propionyl derivative of SF2587 [Compound 3: the compound of formula (I) wherein X = $CH_3CH_2CO$-; Y = H ]
EI-MS: m/z 645 ($M^+$)

## EXAMPLE 4

### Synthesis of 3′,11-O-dipropionyl SF2587 (Compound 4)

In 10 mℓ of pyridine was suspended 500 mg of SF2587, and 5 mℓ of propionic anhydride was added dropwise to the suspension while stirring under ice-cooling. The mixture was stirred under ice-cooling for 30 minutes and then at 20 °C for 36 hours. The reaction mixture was poured into 80 mℓ of ice-water and extracted twice with 50 mℓ portions of chlorofrom. The chloroform layer was separated, washed three times with 100 mℓ portions of water and then with 100 mℓ of a saturated sodium chloride aqueous solution, and dried over anhydrous sodium sulfate. The sodium sulfate was removed by filtration, and the resulting

chloroform solution was concentrated under reduced pressure to obtain a yellow oily substance. The oily substance was dried under reduced pressure overnight and purified by silica gel column chromatography using a toluence/acetone (20:1 followed by 10:1, and finally followed by 5:1 by volume) as an eluent system to obtain 460 mg of a crude product. Recrystallization of the crude product from a chloroform/methanol (20:1) mixed solution yielded 355 mg of a 3′,11-O-dipropionyl derivative of SF2587 [Compound 4: the compound of formula (I) wherein X = $CH_3CH_2CO$; Y = $CH_3CH_2CO$- ] as a yellow needle-like crystal.

Melting Point: 209-212 °C
[α] = +56.0° (c=0.1, chloroform)
EI-MS: m/z 701 ($M^+$)
UV λ nm(ε): 240(31230), 261(29790), 362(7470)
IR ν $cm^{-1}$: 3420, 1760, 1730, 1670, 1650, 1580
$^1$H-NMR ($CDCl_3$, ppm): 8.21 d, 8.05 d, 7.99 s, 6.47 s, 5.32 m, 5.25 d, 4.32 dq, 3.53 d, 3.13 dd, 2.97 s, 2.86 dd, 2.48 q, 2.46 q, 2.33 dd, 2.28 s, 1.90 s, 1.56 dd, 1.43 d, 1.40 d, 1.39 t 1.23 t, 1.05 s
$^{13}$C-NMR ($CDCl_3$, ppm): 181.9, 180.5, 178.9, 173.7, 172.6, 65.6, 155.7, 148.5, 146.3, 145.0, 135.3, 132.6, 132.4, 126.4, 125.7, 125.3, 125.2, 121.5, 110.7, 75.1, 70.6, 64.3, 63.4, 58.1, 57.3, 55.6, 51.6, 42.6, 38.8, 27.9, 27.9, 23.9, 17.2, 14.7, 14.2, 14.1, 9.3, 8.7

## EXAMPLE 5

### Synthesis of 3′-O-Butyryl SF2587 (Compound 5)

In 5 mℓ of n-butyric anhydride was suspended 100 mg of SF2587, and 70.6 mg of anhydrous sodium acetate was added thereto at 20 °C while stirring, followed by stirring at 20 °C for 27 hours. The reaction mixture was poured into 50 mℓ of ice-water, neutralized with a 0.1N sodium hydroxide aqueous solution, and extracted twice with 25 mℓ portions of chlorofrom. The chlorofrom layer was separated and dried over anhydrous sodium sulfate. The sodium sulfate was removed by filtration, and the resulting chlorofrom solution was concentrated under reduced pressure to obtain 3.8 g of an oily substance. To the product was added 20 mℓ of n-hexane, and the supernatant was removed by filtration to obtain 129.6 mg of an orange precipitate. The precipitate was then subjected to silica gel column chromatogtaphy using a chloroform/methanol (10:1 by volume) mixture as a developing solvent ot obtain 78.8 mg of a crude product. The crude product was purified by gel chromatography using Sephadex LH-20 and a chloroform/methanol (1 : 9 by volume) mixture as an eluent. The resulting fraction was crystallized from chloroform/methanol (20:1) to obtain 58.7 mg of a 3′-O-butyryl derivative of SF2587 [Compound 5: the compound of formula (I) wherein X = $CH_3CH_2CO$-; Y = H] as an orange flaky crystal.

Melting Point: 194-196 °C
EI-MS: m/z 659 ($M^+$)
$^1$H-NMR ($CDCl_3$, ppm): 8.03 s, 7.92 d, 7.82 d, 6.45 s, 5.52 d, 5.20 d, 4.30 dd, 3.37 d, 3.14 dq, 2.97 s, 2.91 dd, 2.45 dd, 2.40 s, 2.31 t, 1.94 s, 1.75 tq, 1.67 dd, 1.47 d, 1.46 d, 1.04 s, 1.02 t

## EXAMPLE 6

### Synthesis of 3′-11-O-dibutyryl SF2587 (Compound 6)

In 4 mℓ of pyridine was suspended 100 mg of SF2587, and 2 mℓ of n-butyric anhydride was added thereto dropwise while stirring under ice-cooling. After the addition, the stirring under ice-cooling was continued for 30 minutes, and the mixture was further stirred at 20 °C for 40 hours. The reaction mixture was poured into 20 mℓ of ice-water and then extracted twice with 20 mℓ portions of chloroform. The chloroform layer was separated, successively washed with 20 mℓ portions of water (three times) and then 20 mℓ of a saturated sodium chloride aqueous solution, and dried over anhydrous sodium sulfate. The sodium sulfate was removed by filtration, and the resulting chlorofrorm solution was concentrated under reduced pressure to obtain a yellow oily substance. After being dried under reduced pressure overnight, the residue was passed through a column of silica gel, washed with toluene, and eluted successively with 20:1, 10:1, and 5:1 mixtures of toluene and acetone to obtain 105 mg of a yellow 3′,11-O-dibutyryl derivative of SF 2587 [Compound 6: the compound of formula (I) wherein X = $CH_3CH_2CH_2CO$-; Y = $CH_3CH_2CH_2CO$-].

[α] = +56.7° (c=0.1, chloroform)
EI-MS: m/z 729 ($M^+$)
UV λ nm(ε): 240(44430), 263(42170), 362(10570)
IR ν $cm^{-1}$: 3430, 1760, 1730, 1670, 1650, 1590

$^1$H-NMR (CDCℓ$_3$, ppm): 8.21 d, 8.05 d, 7.98 s, 6.45 s, 5.32 m, 5.23 d, 4.30 dq, 3.47 d, 3.13 dq, 2.96 s, 2.86 dd, 2.42 t, 2.41 t, 2.38 dd, 2.27 s, 1.92 tq, 1.91 s, 1.74 tq, 1.56 dd, 1.44 d, 1.41 d, 1.15 t, 1.02 s, 1.01 t
$^{13}$C-NMR (CDCℓ$_3$, ppm): 181.9, 180.5, 178.9, 172.8, 172.0, 165.7, 155.7, 148.5, 146.4, 145.1, 135.3, 132.6, 132.6, 126.4, 125.7, 125.4, 125.2, 121.5, 110.5, 75.4, 70.5, 64.2, 63.6, 57.9, 57.4, 55.6, 51.6, 42.9, 39.2, 36.6, 36.3, 23.9, 18.6, 18.0, 17.2, 14.7, 14.1, 14.0, 13.9, 13.7

## EXAMPLE 7

### Synthesis of 3′-O-isobutyryl SF2587 (Compound 7)

In 2 mℓ of isobutyric anhydride was suspended 20.7 mg of SF2587, and 14.4 mg of anhydrous sodium acetate was added to the suspension while stirring at 20 °C, followed by stirring at that temperature for 43 hours. The reaction mixture was poured into 20 mℓ of ice-water, neutralized with a 0.1N sodium hydroxide aqueous solution, and extracted twice with 10 mℓ portions of chloroform. The chloroform layer was washed successively with 20 mℓ of water and 20 mℓ of saturated sodium sulfate. After removing the sodium sulfate by filtration, the resulting chloroform solution was concentrated under reduced pressure to obtain 659.6 mg of an oily substance. To the product was added 20 mℓ of n-hexane, and the supernatant was separated by filtration to recover 19.7 mg of an orange precipitate. The precipitate was subjected to silica gel column chromatography using a 10:1 (by volume) mixture of chloroform and methanol as a developing solution to obtain 5.1 mg of an orange 3′-O-isobutyryl derivative of SF2587 [Compound 7: the compound of formula (I) wherein X = $(CH_3)_2CHCO$-; Y = H)
EI-MS: m/z 659 ($M^+$).

## EXAMPLE 8

### Synthesis of 3′,11-O-diisobutyryl SF2587 (Compound 8)

In 4 mℓ of pyridine was suspended 200 mg of SF2587, and 2 mℓ of isobutyric anhydride was added thereto dropwise while stirring under ice-cooling. Thereafter, the stirring under ice-cooling was continued for 30 minutes, and the mixture was further stirred at 20 °C for 43 hours. To the mixture was added 10 mg of demethylaminopyridine, followed by stirring at 20 °C for 6.5 hours. The reaction mixture was poured into 20 mℓ of ice-water and extracted twice with 25 mℓ portions of chloroform. The chloroform layer was separated, washed successively with 30 mℓ portions of water (three times) and 30 mℓ of a saturated sodium chloride aqueous solution, and dried over anhydrous sodium sulfate. After removing the sodium sulfate by filtration, the resulting chloroform solution was concentrated under reduced pressure to obtain a yellow oily substance. The crude product was dried under reduced pressure overnight and then purified by silica gel column chromatography using chloroform followed by a 100:1 (by volume) mixture of chloroform and methanol to obtain 182.6 mg of a yellow 3′,11-O-diisobutyryl derivative of SF2587 substance [Compound 8: the compound of formula (I) wherein X = $(CH_3)_2CHCO$-; Y = $(CH_3)_2CHCO$-].
EI-MS: m/z 729 ($M^+$).

## EXAMPLE 9

### Synthesis of 3′,11-O-divaleryl SF2587 (Compound 9)

In 8 mℓ of pyridine was suspended 200 mg of SF2587, and 4 mℓ of n-valeric anhydride was added thereto dropwise while stirring under ice-cooling. Thereafter, the stirring under ice-cooling was continued for 30 minutes, and the mixture was further stirred at 20 °C for 47 hours. The reaction mixture was poured into 30 mℓ of ice-water and then extracted twice with 30 mℓ portions of chloroform. The chloroform layer was separated, washed three times with 30 mℓ portions of water and then with 30 mℓ of a saturated sodium chloride aqueous solution, and dried over anhydrous sodium sulfate. After removintg the sodium sulfate by filtration, the resulting chloroform solution was concentrated under reduced pressure to obtain 2.40 g of an oily substance. To the product was added 30 mℓ of n-hexane, and the supernatant was separated by filtration to recover 301.8 mg of an orange precipitate. The precipitate was purified by silica gel coulumn chromatography using chloroform as a solvent to obtain 154.5 mg of a yellow 3′,11-O-divaleryl SF2587 [Compound 9: the compound of formula (I) wherein X = $CH_3(CH_2)_3CO$-; and Y = $CH_3(CH_2)_3CO$-].
EI-MS: m/z 757 (M + )

EXAMPLE 10

Synthesis of 11-O-Butyryl SF2587 (Compound 10)

Two milliliters of pyridine were added to a solution of 102 mg of SF2587 in 5 mℓ of dichloromethane, and 5 mℓ of a dichloromethane solution of n-butyryl chloride was added thereto at room temperature while stirring. To the mixture was further added 8 mg of dimethylaminopyridine, followed by stirring at 25 °C for 24 hours. The reaction mixture was poured into 20 mℓ of ice-water and extracted twice with 10 mℓ portions of dichloromethane. The dichloromethane layer was separated, washed twice with 10 mℓ portions of water and then with 20 mℓ of a saturated sodium chloride aqueous solution, and dried over anhydrous sodium sulfate. The sodium sulfate was removed by filtration, and the resulting dichloromethane solution was concentrated under reduced pressure to obtain a yellowish brown oily substance. The substance was dried under reduced pressure overnight and purified by silica gel column chromatography using chloroform followed by a 20:1 (by volume) mixture of chloroform and methanol as solvents to obtain 35.9 mg of a yellow 11-O-butyryl derivative of SF2587 [Compound 10: the compound of formula (I) wherein X = H; and Y = $CH_3CH_2CH_2CO$-].

EI-MS: M/Z 659 (M+)

$^1$H-NMR ($CDCℓ_3$, ppm): 8.18 d, 7.96 s, 7.95 d, 6.42 s, 5.37 m, 4.19 m, 3.75 d, 3.37 d, 3.10 tq, 2.93 s, 2.84 dd, 2.40 s. 2.28 t, 1.94 m, 1.89 s, 1.87 m, 1.64 tq, 1.41 d, 1.37 d, 1.12 s, 0.95 t

REFERENCE EXAMPLE

As a seed culture medium, a medium composed of 2.0% starch, 1.0% glucose, 0.6% wheat germ, 0.5% polypeptone, 0.3% yeast extract, 0.2% soybean meal, and 0.2% calcium carbonate was used.

As a production medium, a medium composed of 2.0% maltose syrup, 0.15% soyvbean oil, 1.0% soybean meal, 0.5% Pharmamedia, 0.25% distiller's soluble, 0.1% calcium carbonate, 0.0005% ferrous sulfate ($7H_2O$), 0.00005% cobalt chloride ($6H_2O$) and 0.00005% nickel chloride was ($6H_2O$) was used. Each medium was adjusted to pH 7.0 prior to sterilization.

A 100 mℓ Erlenmeyer flask containing 20 mℓ of the above seed culture medium was sterilized at 120 °C for 30 minutes and then, inoculated with 2-3 loopfuls of Streptomyces sp. SF2587 (FERM BP-2244) grown on an agar slant. The incubation was performed under shaking at 28 °C for 3 days to give a first seed culture. Then, a 500 mℓ Erlenmeyer flask containing 80 mℓ of the same seed culture medium as above was sterilized at 120 °C for 30 minutes and, after cooling, inoculated with 2.4 mℓ of the above-prepared first seed culture. The incubation was carried out under shaking at 28 °C for one day to give a second seed culture.

Four jar fermenters of 50-liter capacity, each containing 35 liters of the production medium, which was previously sterilized at 120 °C for 30 minutes, were inoculated with 300 mℓ portions of said second seed culture. The incubation was carried out at 28 °C for 4 days under aeration (20 ℓ/min.) and stirring (250 rpm).

After completion of incubation, the culture broth was filtered with the aid of diatomaceous earch to provide a cell-containing solid fraction.

This solid fraction was extracted with 60 ℓ of 67% acetone-water at 20 °C with stirring and, then, filtered to remove the solid matter. This extract was distilled to remove acetone under reduced pressure and 10 ℓ of the resulting concentrate was extracted with 15 ℓ of ethyl acetate. The ethyl acetate layer was dehydrated over anhydrous sodium sulfate and concentrated under reduced pressure to give 8.98 g of oil. This oil was evenly mixed with 9 g of diatomaceous earth and dried under reduced pressure for 16 hours. It was then applied to a column in which 400 mℓ of silica gel C-200 (Wako Pure Chemical Industries) was packed with chloroform. The column was washed with chloroform and chloroform/methanol mixtures (100:1, 50:1, 20:1 and 10:1) in the order mentioned and finally elution was carried out with chloroform/methanol (5:1). The eluate was subjected to the cytotoxitity assay (MTT assay) against mouse leukemia cells (P-388) as described in Test Example 1 below and the fractions rich in cytotoxicity, which show 50% growth inhibition against P-388 cells when diluted 1:200, were pooled and concentrated to dryness under reduced pressure to provide 235 mg of oil. This oil was dissolved in a small amount of methanol and applied to a column in which 300 mℓ of Toyopearl HW-40 (Tosoh Corporation) was packed with methanol and elution was carried out with methanol. The eluate was subjected to the cytotoxicity assay using mouse leukemia P-388 cells and the active fractions, which show 100% growth inhibition against P-388 cells when diluted 1:122,000, were pooled, concentrated under reduced pressure and allowed to stand at 5 °C for 16 hours. As a result, SF2587 substance separated out as a yellow precipitate. This precipitate was recovered by filtration and dried under reduced pressure at 40 °C for 16 hours, whrereby 3.0 mg purified SF2587 was

obtained as a yellow powder.

## TEST EXAMPLE 1

### Cytotoxicity on P-388 Cells

Cytotoxicity of the compounds according to the present invention was determined by MTT assay in the following manner.

Mouse leukemia P-388 cells were suspended in RPMI medium supplemented with 10% fetal calf serum and this suspension was used to inoculate the 96-well microplate to give a cell concentration of $2 \times 10^3$ cells/well. The compound shown in Table 2 was dissloved in RPMI medium and add to each well for the test group, while no compound was added to wells of the control group. Incubation was carried out at 37°C for 72 hours at 5% $CO_2$. Then, MTT (3-(4,5-dimethylthiazol-2-yl)-2,5-diphenyl tetrazolium bromide) was added to each well to cause formation of formazan. The resulting formazan was dissolved by adding sodium dodecyl sulfate. Absorbance at 577-630 nm of each well was measured by the ELISA analyzer and 50% ihibitory concentration ($IC_{50}$) of each compound was calculated from the absorbance ratio of the test group to the control group. The results are shown in Table 2.

Table 2

| Compound No. | $IC_{50}$ (ng/mℓ) |
|---|---|
| 1 | 19.4 |
| 2 | 23.1 |
| 3 | 19.9 |
| 4 | 18.1 |
| 5 | 17.8 |
| 6 | 23.1 |
| 7 | 55.2 |
| 8 | 23.1 |
| 9 | 34.3 |
| 10 | 16.0 |

## TEST EXAMPLE 2

### Antitumor Activity

Antitumor activity of hydrochlorides of the compounds of the present invention was determined in the following manner.

Mouse leukemia P-388 cells maintained in the abdominal cavity of $BDF_1$ mouse were taken out and were suspended in physiological saline to give a cell concnetration of $1 \times 10^6$ cells/0.2 mℓ. The cell suspension (0.2 mℓ) was transplanted to the abdominal cavity of 5-week-old male $BDF_1$ mice. The compounds of the present invention as shown in Table 3 was dissolved in 0.2 mℓ of physiological saline and was intraperitoneally administered to the mouse 24 hours after the transplantation of P-388 cells. No compound was administered to the mice of the control group. The T/C% value was calculated in accordance with the following formula.

$$T/C(\%) = \frac{\text{Survival days of mice of the test group}}{\text{Survival days of mice of the control group}} \times 100$$

The results are shown in Table 3.

Table 3

| Compound No. | Dose (mg/kg) | Anti-P388 Activity (T/C%) |
|---|---|---|
| 1 | 1.0 | 144 |
| 1 | 0.5 | 134 |
| 1 | 0.25 | 124 |
| 2 | 14 | 70 |
| 2 | 7 | >200 |
| 2 | 3.5 | 163 |
| 4 | 25 | 71 |
| 4 | 12.5 | >449 |
| 4 | 6.25 | 217 |
| 4 | 3.13 | 133 |
| 4 | 1.56 | 130 |
| 5 | 5 | 72 |
| 5 | 2.5 | 186 |
| 5 | 1.25 | 158 |
| 6 | 20 | >485 |
| 6 | 10 | 186 |
| 6 | 5 | 144 |
| 6 | 2.5 | 113 |
| 6 | 1.25 | 96 |
| 8 | 40 | 69 |
| 8 | 20 | >488 |
| 8 | 10 | 175 |
| 8 | 5 | 144 |
| 8 | 2.5 | 113 |
| 9 | 40 | 227 |
| 9 | 20 | 189 |
| 9 | 10 | 147 |
| 9 | 5 | 117 |
| 9 | 2.5 | 96 |

TEST EXAMPLE 3

Acute Toxicity

The hydrochlorides of the compounds of the present invention as shown in Table 4 were intraperitoneally or intravenously administered to mice and acute toxicity ($LD_{50}$) of each compound was measured. The results are shown in Table 4.

Table 4

| Compound No. | Injection route | $LD_{50}$ (mg/kg) |
|---|---|---|
| 2 | i.p. | 16 |
| 4 | i.p. | 40 |
| 6 | i.p. | 120 |
| 2 | i.v. | 23.5 |

As demonstrated above, the SF2587 substance derivative according to the present invention has reduced acute toxicity and enhanced antitumor activity as compared with the starting SF2587 and is thus promising as an anticancer agent.

FORMULATION EXAMPLE 1

Two grams of Compound 6 obtained in Example 6, an adequate amount of a peanut oil, and 1 g of benzyl alcohol were mixed, and a peanut oil was added thereof to make 100 mℓ. The solution was sealed in ampules by 1 mℓ portions under sterile conditions.

FORMULATION EXAMPLE 2

Two grams of a hydrochloride of Compound 2 obtained in Example 2 were dissolved in 400 mℓ of physiological saline. The solution was sterilized and sealed in ampules by 1 mℓ portions under sterile conditions.

FORMULATION EXAMPLE 3

Twenty grams of Compound 6 obtained in Example 6 and 65 g of polyethylene glycol (Macrogol 400) were mixed to form a uniform solution. Separately, a gelatin solution consisting of 45 g of gelatin, 10 g of glycerin, 5 g of D-sorbitol, 0.2 g of ethyl p-hydroxybenzoate, 0.1 g of propyl p-hydroxybenzoate, and 0.2 g of titanium oxide was prepared. Soft capsules each containing 200 mg of contents were produced using the above-prepared gelatin solution as a capsule-forming material according to a manual plate punching method.

**Claims**

1. A compound represented by formula (I):

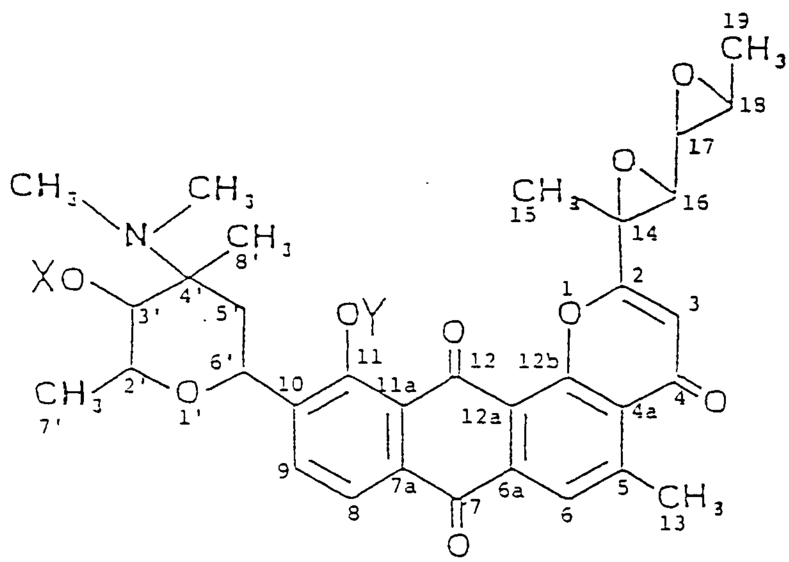

(I)

wherein X and Y represents a hydrogen atom or a group RCO-, wherein R represents an alkyl group having from 1 to 4 carbon atoms, provided that X and Y do not simultaneously represent a hydrogen atom, or a pharmaceutically acceptable salt thereof.

2. The compound of claim 1, wherein the group RCO- is selected from an n-propionyl group, an n-butyryl group, an isobutyryl group and an n-valeryl group.

3. An anticancer agent containing a compound represented by formula (I):

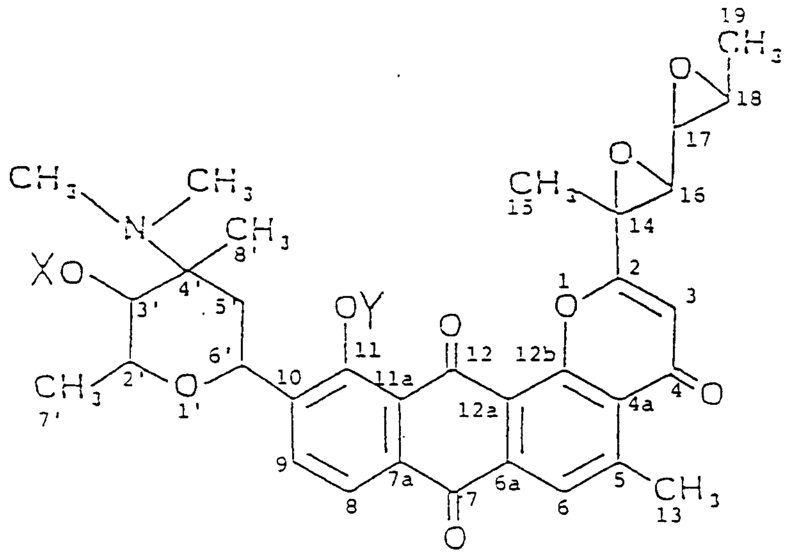

(I)

wherein X and Y represents a hydrogen atom or a group RCO-, wherein R represents an alkyl group having from 1 to 4 carbon atoms, provided that X and Y do not simultaneously represent a hydrogen atom, or a pharmaceutically acceptable salt thereof as an active ingredient, and a pharmaceutically acceptable carrier.

4. The anticancer agent of claim 3, wherein the group RCO- is selected from a n-propionyl group, an n-butyryl group, an isobutyryl group and an n-valeryl group.

5. A process for preparing a compound represented by formula (I):

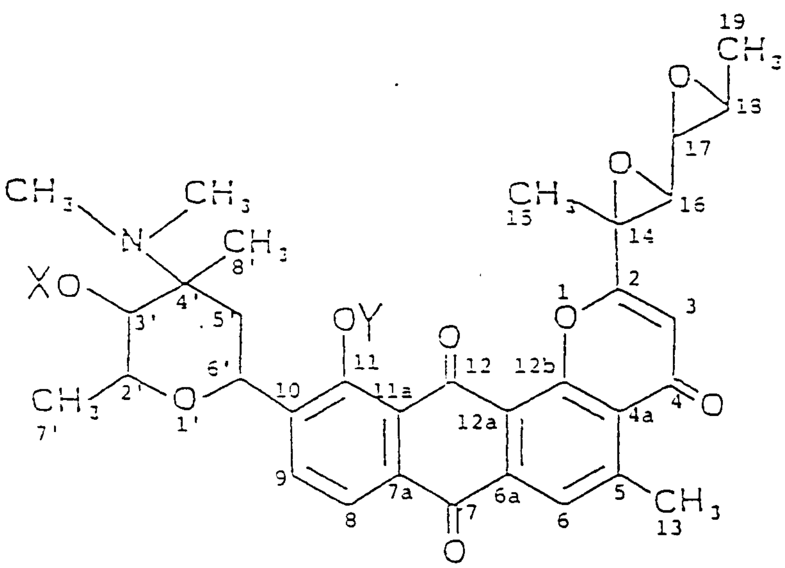

(I)

wherein X and Y represents a hydrogen atom or a group RCO-, wherein R represents an alkyl group having from 1 to 4 carbon atoms, provided that X and Y do not simultaneously represent a hydrogen atom, which comprises reacting a substance represented by the formula (II)

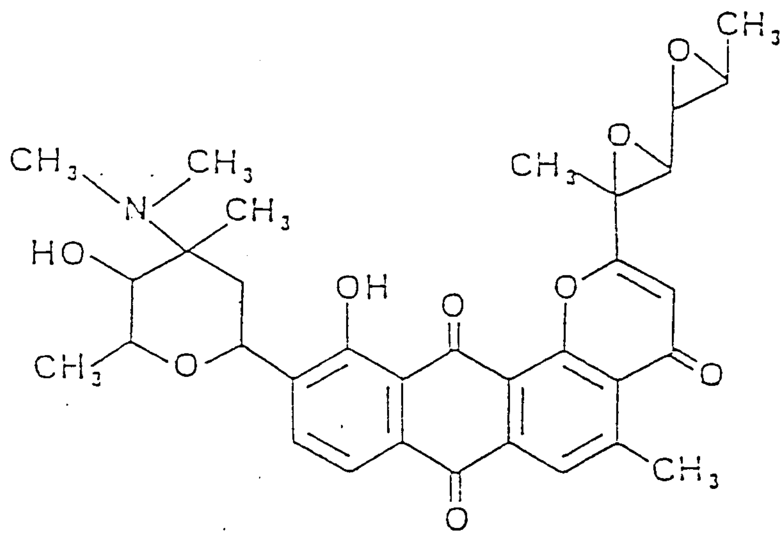

(II),

with an acylating agent in the presence of a base.

6. The process of claim 5, wherein said acylating agent is selected from acetic anhydride, acetyl chloride, propionic anhydride, propionyl chloride, n-butyric anhydride, n-butyryl chloride isobutyryl chloride, isobutyric anhydride, n-valeryl chloride and n-valeric anhydride.

7. The process of claim 5, wherein said base is selected from sodium acetate, pyridine, dimethylaminopyridine, and triethylamine.

8. The use of a compound represented by formula (I):

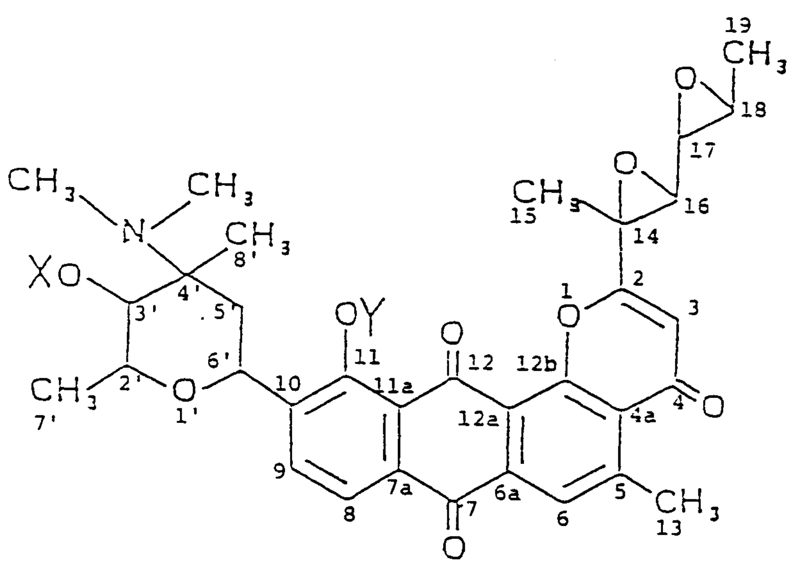

(I)

wherein X and Y represents a hydrogen atom or a group RCO-, wherein R represents an alkyl group having from 1 to 4 carbon atoms, provided that X and Y do not simultaneously represent a hydrogen atom, or a pharmaceutically acceptable salt thereof for the manufacture of a medicament for the treatment of cancer.

## Patentansprüche

1. Verbindung gemäß Formel (I)

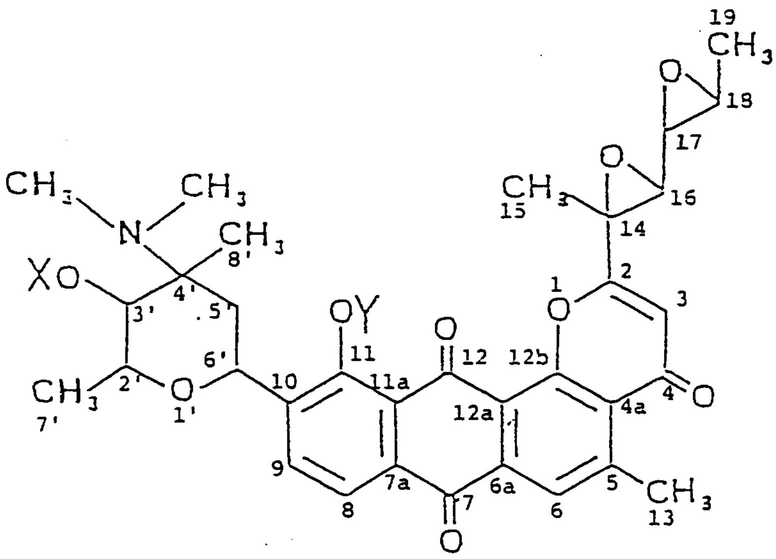

(I)

in der X und Y Wasserstoff oder eine RCO-Gruppe sind, wobei R für eine Alkylgruppe mit 1 bis 4 Kohlenstoffatomen steht, vorausgesetzt, daß X und Y nicht gleichzeitig Wasserstoff sind, sowie deren pharmazeutisch verträgliches Salz.

2. Verbindung nach Anspruch 1, wobei die Gruppe RCO eine n-Propionylgruppe, n-Buturylgruppe, Isobutyrylgruppe und/oder n-Valerylgruppe ist.

3. Antitumorsubstanz mit Gehalt an einer Verbindung der Formel (I):

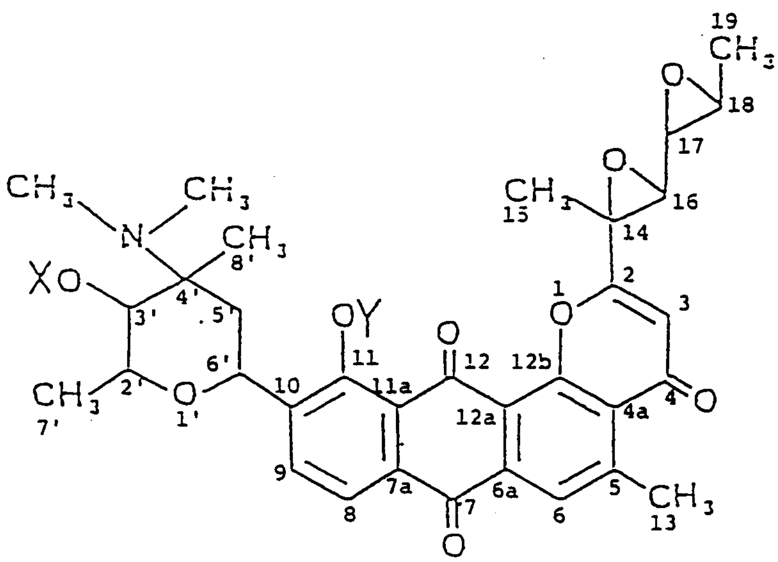

(I)

in der X und Y Wasserstoff oder eine RCO-Gruppe sind, wobei R für eine Alkylgruppe mit 1 bis 4 Kohlenstoffatomen steht, vorausgesetzt, daß X und Y nicht gleichzeitig Wasserstoff sind, sowie deren pharmazeutisch verträgliches Salz als aktiver Wirkstoff und einem pharmazeutisch verträglichen Träger.

4. Antitumorsubstanz nach Anspruch 3, wobei die Gruppe RCO eine n-Propionylgruppe, n-Butyrylgruppe, Isobutyrylgruppe und/oder n-Valerylgruppe ist.

**5.** Verfahren zur Herstellung einer Verbindung gemäß Formel (I):

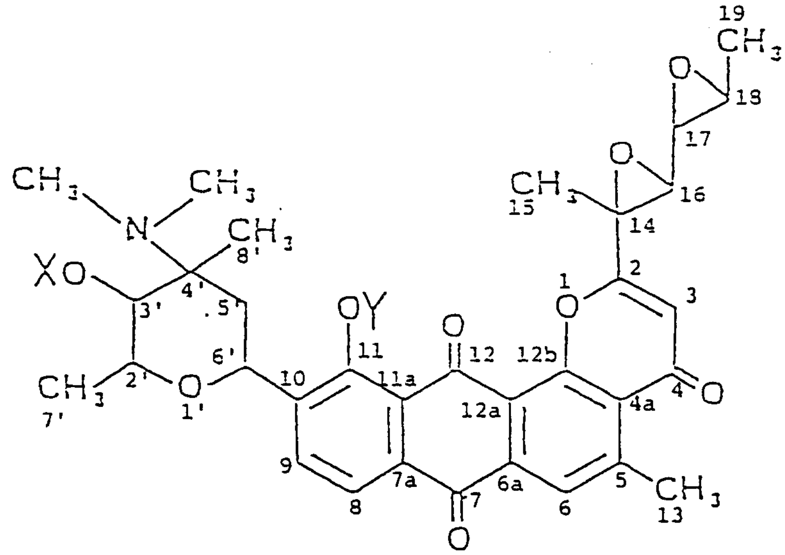

(I)

in der X und Y Wasserstoff oder eine RCO-Gruppe sind, wobei R für eine Alkylgruppe mit 1 bis 4 Kohlenstoffatomen steht, vorausgesetzt, daß X und Y nicht gleichzeitig Wasserstoff sind, wobei eine Substanz gemäß Formel (II)

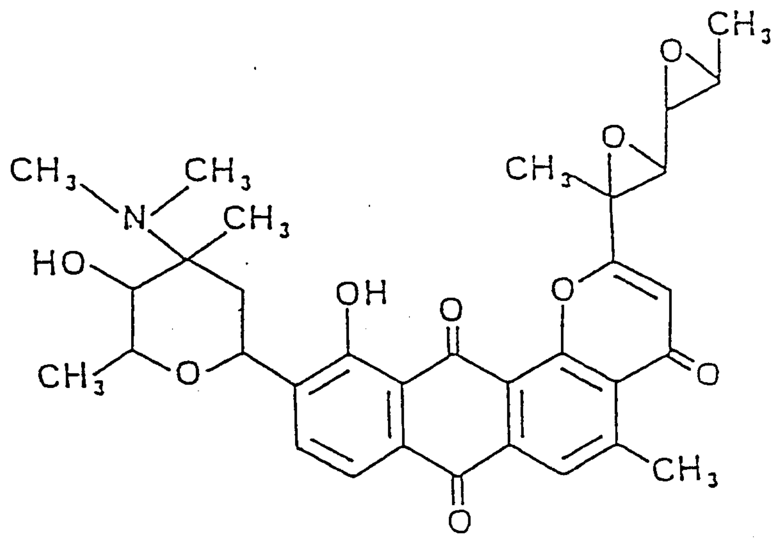

(II),

mit einem Acylierungsmittel in Gegenwart einer Base umgesetzt wird.

**6.** Verfahren nach Anspruch 5, wobei das Acylierungsmittel Essigsäureanhydrid, Acetylchlorid, Propionsäureanhydrid, Propionylchlorid, n-Buttersäureanhydrid, n-Butyrylchlorid, Isobutyrylchlorid, Isobuttersäureanhydrid, n-Valerylchlorid und/oder n-Valerinsäureanhydrid ist.

**7.** Verfahren nach Anspruch 5, wobei die Base Natriumacetat, Pyridin, Dimethylaminpyridin und/oder Trietylamin ist.

**8.** Verwendung einer Verbindung gemäß Formel (I),

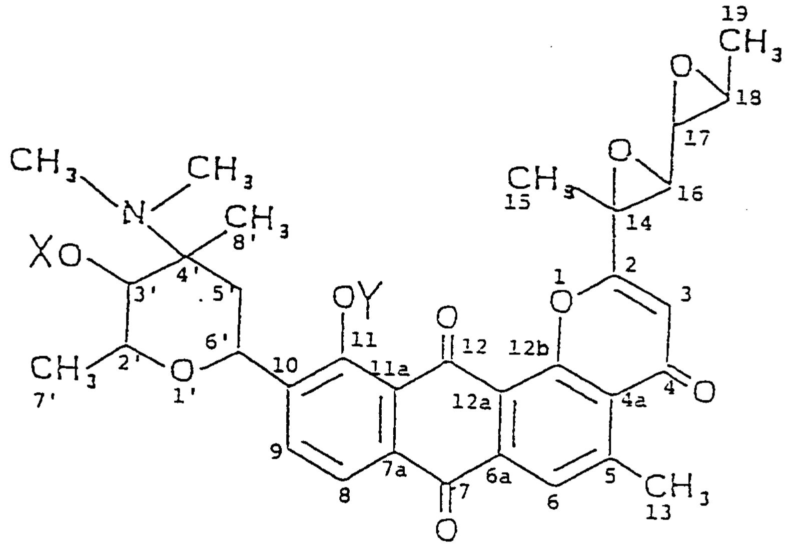

(I)

in der X und Y Wasserstoff oder eine RCO-Gruppe sind, wobei R für eine Alkylgruppe von 1 bis 4 Kohlenstoffatomen steht, vorausgesetzt, daß X und Y nicht gleichzeitig Wasserstoff sind, oder deren pharmazeutisch verträgliches Salz zur Herstellung eines Arzneimittels für die Krebsbehandlung.

## Revendications

**1.** Composé représenté par la formule (I) :

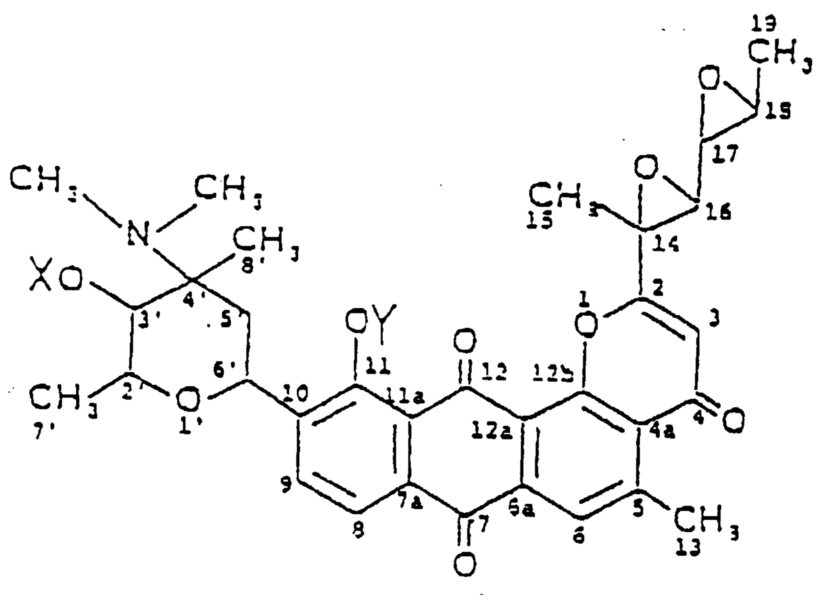

(I)

où X et Y représentent un atome d'hydrogène ou un groupe RCO-, dans lequel R représente un groupe alkyle ayant de 1 à 4 atomes de carbone, pourvu que X et Y ne représentent pas simultanément un atome d'hydrogène, ou un de ses sels acceptable pharmaceutiquement.

**2.** Composé selon la revendication 1, dans lequel le groupe RCO- est choisi parmi un groupe n-propionyle, un groupe n-butyryle, un groupe isobutyryle et un groupe n-valéryle.

3. Agent anticancer contenant un composé représenté par la formule (I) :

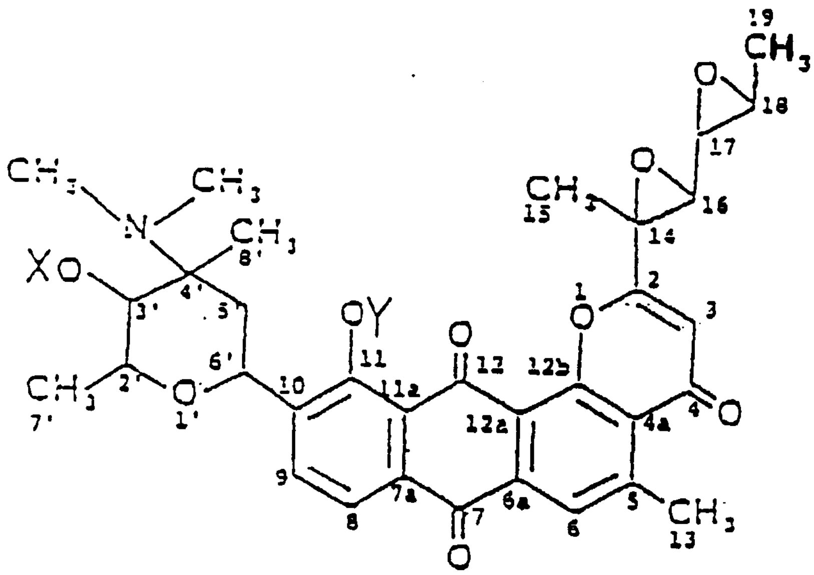

(I)

dans laquelle X et Y représentent un atome d'hydrogène ou un groupe RCO-, dans lequel R représente un groupe alkyle ayant de 1 à 4 atomes de carbone, pourvu que X et Y ne représentent pas simultanément un atome d'hydrogène, ou un de ses sels pharmaceutiquement acceptable comme ingrédient actif, et un support pharmaceutiquement acceptable.

4. Agent anticancer selon la revendication 3, dans lequel le groupe RCO- est choisi parmi un groupe n-propionyle, un groupe n-butyryle, un groupe isobutyryle et un groupe n-valéryle.

5. Procédé de préparation d'un composé représenté par la formule (I) :

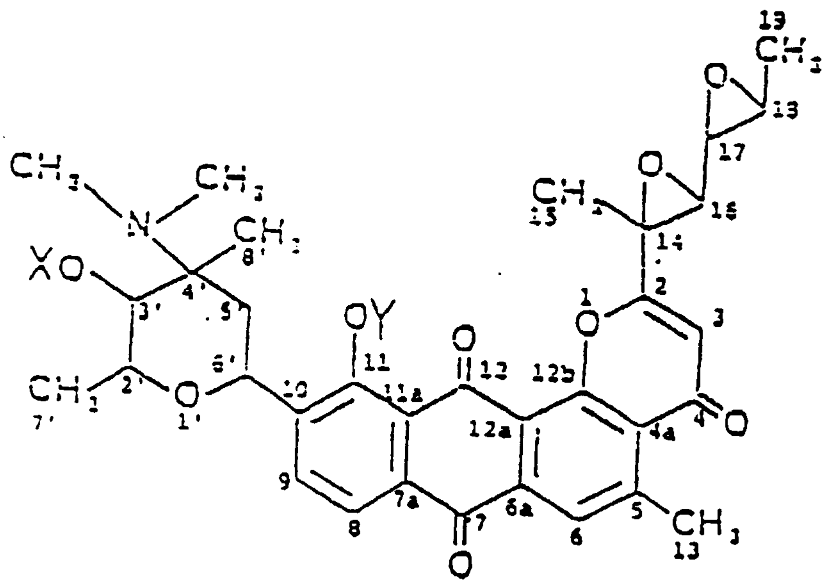

(I)

dans laquelle X et Y représentent un atome d'hydrogène ou un groupe RCO-, dans lequel R représente un groupe alkyle ayant de 1 à 4 atomes de carbone , pourvu que X et Y ne représentent pas simultanément un atome d'hydrogène, qui comprend la réaction d'une substance représentée par la formule (II) :

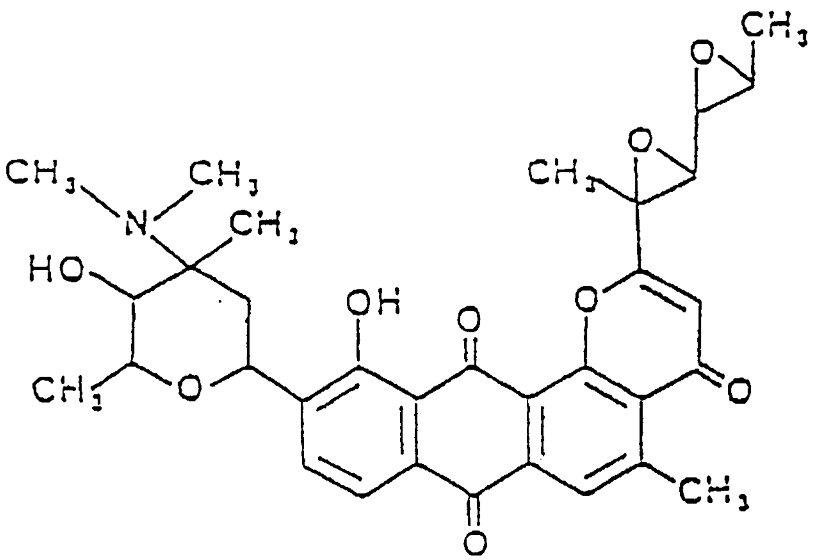

(II)

avec un agent d'acylation en présence d'une base.

6. Procédé selon la revendication 5, dans lequel ledit agent d'acylation est choisi parmi anhydride acétique, chlorure d'acétyle, anhydride propionique, chlorure de propionylee, anhydride n-butyrique, chlorure de n-butyryle, chlorure d'isobutyryle, anhydride isobutyrique, chlorure de n-valéryle et anhydride n-valérique.

7. Procédé selon la revendication 5, dans lequel ladite base est choisie parmi acétate de sodium, pyridine, diméthylaminopyridine, et triéthylamine.

8. Utilisation d'un composé représenté par la formule (I) :

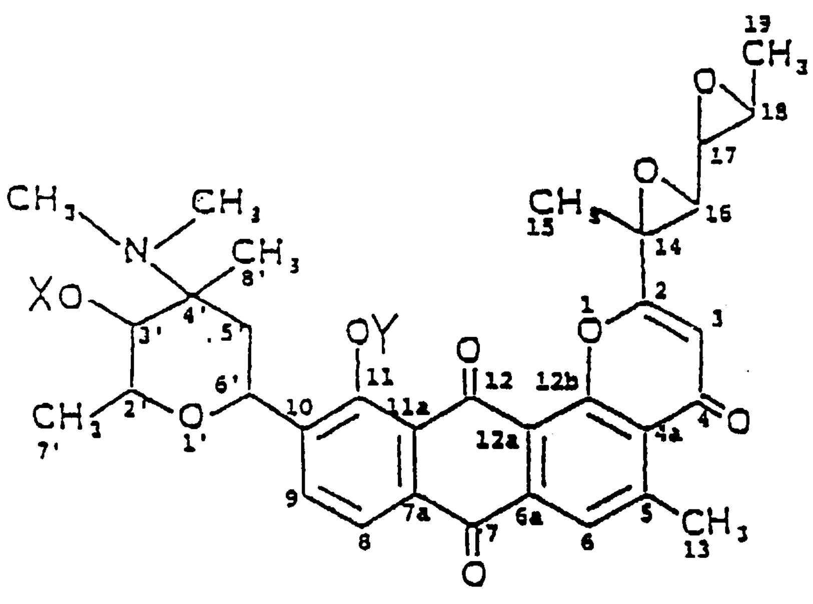

(I)

dans laquelle X et Y représentent un atome d'hydrogène ou un groupe RCO-, dans lequel R représente un groupe alkyle ayant de 1 à 4 atomes de carbone, pourvu que X et X ne représentent pas simultanément un atome d'hydrogène, ou un de ses sels pharmaceutiquement acceptable pour la fabrication d'un médicament pour le traitement du cancer.